(19)

Europäisches
Patentamt
European
Patent Office
Office européen
des brevets

(11)  **EP 4 343 782 A1**

(12)  **EUROPEAN PATENT APPLICATION**

(43) Date of publication:
**27.03.2024  Bulletin 2024/13**

(21) Application number: **23198282.8**

(22) Date of filing: **19.09.2023**

(51) International Patent Classification (IPC):
**G16H 30/40** (2018.01)     **G06N 3/045** (2023.01)
**G16H 50/20** (2018.01)

(52) Cooperative Patent Classification (CPC):
**G16H 50/20; G06N 3/045; G16H 30/40**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **22.09.2022  US 202217934213
22.09.2022  EP 22465552**

(71) Applicant: **Siemens Healthineers AG
91301 Forchheim (DE)**

(72) Inventors:
  • **GULSUN, Mehmet Akif
    Princeton, NJ 08540 (US)**

  • **STOIAN, Diana Ioana
    507220 Tarlungeni, Brasov (RO)**
  • **SINGH, Vivek
    Princeton, NJ 08540 (US)**
  • **SHARMA, Puneet
    Princeton Junction, NJ 08550 (US)**
  • **BERGER, Martin
    91088 Bubenreuth (DE)**

(74) Representative: **Horn Kleimann Waitzhofer
Schmid-Dreyer
Patent- und Rechtsanwälte PartG mbB
Theresienhöhe 12
80339 München (DE)**

(54)  **A MULTI-TASK LEARNING FRAMEWORK FOR FULLY AUTOMATED ASSESSMENT OF
CORONARY ARTERIES IN ANGIOGRAPHY IMAGES**

(57)  Systems and methods for automatic assessment of a vessel are provided. A temporal sequence of medical images of a vessel of a patient is received. A plurality of sets of output embeddings is generated using a machine learning based model trained using multi-task learning. The plurality of sets of output embeddings is generated based on shared features extracted from the temporal sequence of medical images. A plurality of vessel assessment tasks is performed by modelling each of the plurality of sets of output embeddings in a respective probabilistic distribution. Results of the plurality of vessel assessment tasks are output.

FIG 1

100

Receive a temporal sequence of medical images of a vessel of a patient
102

Generate a plurality of sets of output embeddings using a machine learning based model trained using multi-task learning, the plurality of sets of output embeddings generated based on shared features extracted from the temporal sequence of medical images
104

Perform a plurality of vessel assessment tasks by modelling each of the plurality of sets of output embeddings in a respective probabilistic distribution
106

Output results of the plurality of vessel assessment tasks
108

EP 4 343 782 A1

**Description**

TECHNICAL FIELD

The present invention relates generally to fully automated assessment of coronary arteries in angiography images, and in particular to a multi-task learning framework for fully automated assessment of coronary arteries in angiography images.

BACKGROUND

A coronary angiography is a medical procedure to visualize the blood in the coronary arteries. A coronary angiography allows for the assessment of the coronary arteries for diagnostic reporting and intervention planning. Conventionally, machine learning based approaches for coronary artery assessment from a coronary angiography have been proposed. Such conventional approaches train an ensemble of separate machine learning based models to perform each task, such as, e.g., stenosis detection, stenosis grading, and segment classification, and derive the assessment results in a post-processing step. However, such conventional approaches suffer from inconsistency between results of different tasks and of the results of the overall system and propagation of errors introduced in each task. Further, in such conventional approaches, each machine learning based model is trained individually and therefore lacks the interpretability and explainability of results across different machine learning based models.

BRIEF SUMMARY OF THE INVENTION

In accordance with one or more embodiments, systems and methods for automatic assessment of a vessel are provided. A temporal sequence of medical images of a vessel of a patient is received. A plurality of sets of output embeddings is generated using a machine learning based model trained using multi-task learning. The plurality of sets of output embeddings is generated based on shared features extracted from the temporal sequence of medical images. A plurality of vessel assessment tasks is performed by modelling each of the plurality of sets of output embeddings in a respective probabilistic distribution. Results of the plurality of vessel assessment tasks are output.

In one embodiment, the temporal sequence of medical images may be an angiography sequence. The temporal sequence of medical images may have been acquired at a plurality of different acquisition angles.

In one embodiment, the plurality of sets of output embeddings is generated by extracting shared features from the temporal sequence of medical images using an encoder network and decoding the shared features using a plurality of decoders to respectively generate the plurality of sets of output embeddings.

In one embodiment, each of the plurality of sets of output embeddings is modelled in a respective Gaussian process. A confidence measure for the results of the plurality of vessel assessment tasks is determined using the Gaussian process.

In one embodiment, the plurality of vessel assessment tasks comprises localization of a stenosis in the vessel, image-based stenosis grading of a stenosis in the vessel, and/or segment labelling of a stenosis in the vessel.

These and other advantages of the invention will be apparent to those of ordinary skill in the art by reference to the following detailed description and the accompanying drawings.

BRIEF DESCRIPTION OF THE DRAWINGS

Figure 1 shows a method for automatic assessment of a vessel, in accordance with one or more embodiments;

Figure 2 shows a framework of a multi-task AI system for automatic assessment of a vessel, in accordance with one or more embodiments;

Figure 3 shows a framework of a multi-task AI system for automatic detection of a stenosis of a vessel and a confidence measure associated with the detection of the stenosis, in accordance with one or more embodiments;

Figure 4 shows an exemplary output image of a multi-task AI system, in accordance with one or more embodiments;

Figure 5 shows an exemplary artificial neural network that may be used to implement one or more embodiments;

Figure 6 shows a convolutional neural network that may be used to implement one or more embodiments; and

Figure 7 shows a high-level block diagram of a computer that may be used to implement one or more embodiments.

DETAILED DESCRIPTION

The present invention generally relates to methods and systems for a multi-task learning framework for fully automated assessment of coronary arteries in angiography images. Embodiments of the present invention are described herein to give a visual understanding of such methods and systems. A digital image is often composed of digital representations of one or more objects (or shapes). The digital representation of an object is often described herein in terms of identifying and manipulating the objects. Such manipulations are virtual manipulations accomplished in the memory or other circuitry/hardware of a computer system. Accordingly, is to be understood that embodiments of the present invention may be performed within a computer system using data stored within the computer system.

Embodiments described herein provide for a single end-to-end machine learning based network trained using multi-task learning to perform a plurality of vessel assessment tasks for the fully automated assessment of coronary arteries in angiography images. By utilizing a single end-to-end machine learning based network, results between the plurality of vessel assessment tasks are ensured to be consistent. In addition, the single end-to-end machine learning based network can produce meaningful results regardless of the failure of an individual task.

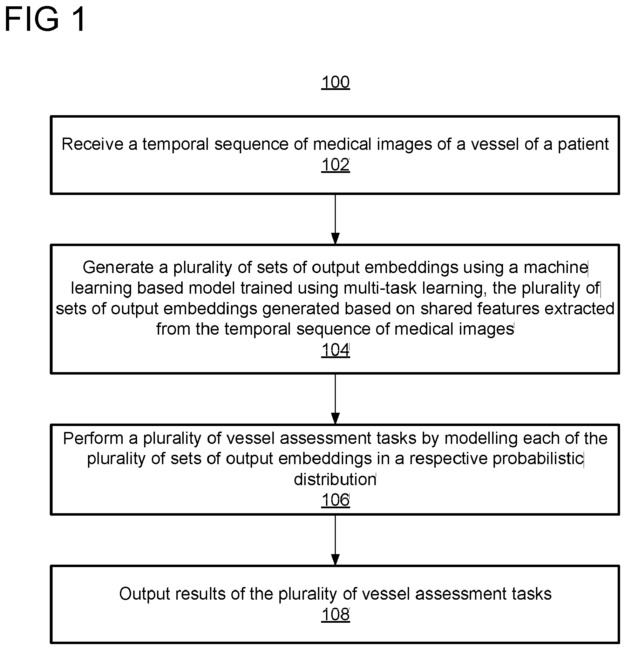

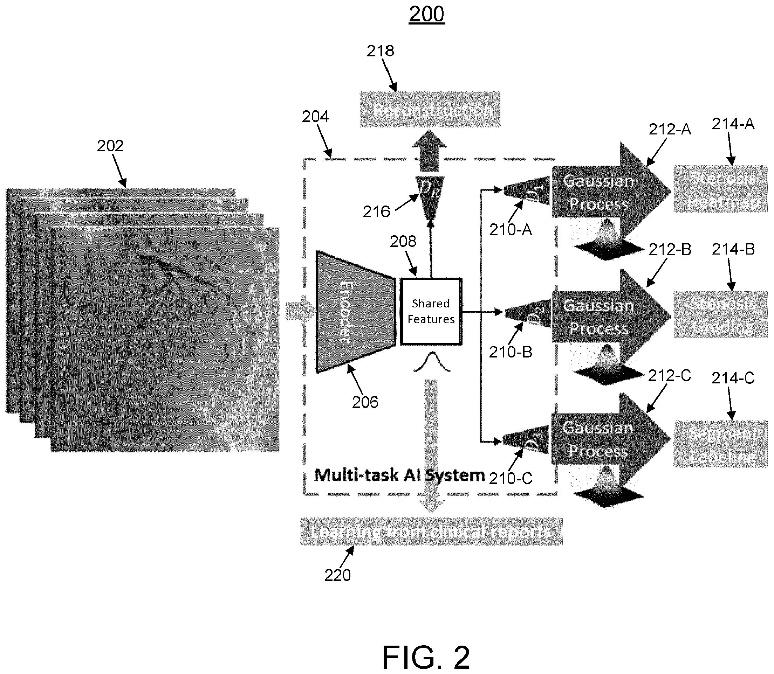

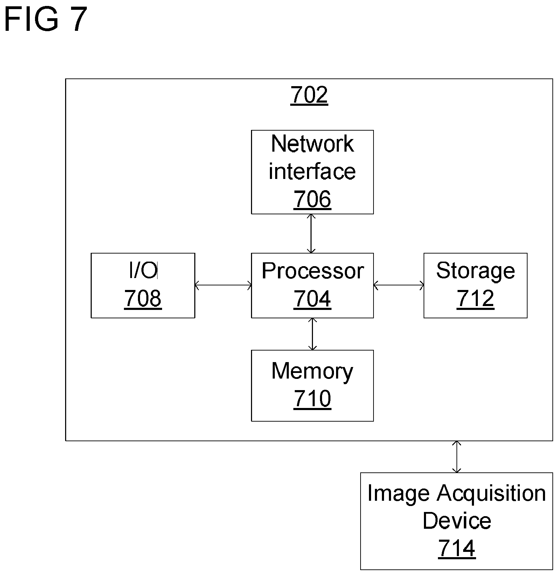

Figure 1 shows a method 100 for automatic assessment of a vessel, in accordance with one or more embodiments. The steps of method 100 may be performed by one or more suitable computing devices, such as, e.g., computer 702 of Figure 7. Figure 2 shows a framework 200 of a multi-task AI (artificial intelligence) system for automatic assessment of a vessel, in accordance with one or more embodiments. Figure 1 and Figure 2 will be described together.

At step 102 of Figure 1, a temporal sequence of medical images of a vessel of a patient is received. The vessel of the patient may be an artery of the patient, a vein of the patient, or any other vessel of the patient. For example, the vessel may be a coronary branch of the patient.

The temporal sequence of medical images is a plurality of medical images of the vessel of the patient acquired over a period of time. In one embodiment, the temporal sequence of medical images is an angiography sequence of images acquired via X-ray coronary angiography at a plurality different acquisition angles. For example, as shown in Figure 2, the temporal sequence of medical images may be angiography sequence 202. However, the temporal sequence of medical images may be of any other suitable modality, such as, e.g., CT (computed tomography), MRI (magnetic resonance imaging), x-ray, US (ultrasound), or any other modality or combination of modalities. The temporal sequence of medical images may comprise 2D (two dimensional) images or 3D (three dimensional) volumes. The temporal sequence of medical images may be received directly from an image acquisition device (e.g., image acquisition device 714 of Figure 7), such as, e.g., a CT scanner, as the images are acquired, or can be received by loading previously acquired images from a storage or memory of a computer system or receiving images from a remote computer system.

At step 104 of Figure 1, a plurality of sets of output embeddings are generated using a machine learning based model trained using multi-task learning. The plurality of sets of output embeddings are generated based on shared features extracted from the temporal sequence of medical images.

In one embodiment, the machine learning based model comprises 1) an encoder for encoding the temporal sequence of medical images into the shared features (i.e., latent features or a latent representation) and 2) a plurality of decoders each for decoding the shared features into respective sets of output embeddings. The shared features are latent features representing the most important features of the temporal sequence of medical images. The output embeddings are outputs of the decoders and may be in any suitable format depending on the vessel assessment task being performed.

In one example, as shown in Figure 2, the machine learning based model is multi-task AI system 204 comprising encoder 206 and a plurality of decoders 210-A, 210-B, and 210-C (collectively referred to as decoders 210). Encoder 206 receives angiography sequence 202 as input and generates shared features 208 as output. Decoders 210 receive shared features 208 as input and respectively generate a set of output embeddings as output. In one embodiment, spectral normalization is used on the encoder 206 and the plurality of decoders 210 to prevent feature collapse (where in- and out-of-distribution input data is mapped to the same location in the feature space). It should be understood that while three decoders 210 are shown in Figure 2, the machine learning based model may comprise any number of decoders each corresponding to a vessel assessment task to be performed.

In one embodiment, the machine learning based network is implemented as a bi-Lipschitz neural network comprising a VAE (variational autoencoder) implementing the encoder and the plurality of decoders. However, the machine learning based model may be any suitable machine learning based model or models for performing the plurality of vessel assessment tasks, such as, e.g., a DNN (deep neural network), a CNN (convolutional neural network), a DI2IN (deep image-to-image network), etc.

The machine learning based model is trained using multi-task learning during a prior offline or training stage based on annotated training data.

In one embodiment, the machine learning based model is trained with decoder 216 to generate a reconstruction 218 of angiography sequence 202. The reconstruction task regularizes the manifold for training to thereby regularize shared features 208.

In one embodiment, the machine learning based model is additionally or alternatively trained using clinical reports, such as, e.g., clinician reports, lab diagnostics reports, etc. For example, as shown in Figure 2, multi-task AI system 204 is trained using clinical reports 220. In one embodiment, the machine learning based model may be first pretrained using training medical images and/or the clinical reports (without annotations) to learn useful shared features and then fine-tuned based on manual annotations to perform various vessel assessment tasks. Once trained, encoder 206 may additionally receive clinical reports as input and encodes the clinical reports into shared features 208. Plurality of decoders 210 decodes the shared features 208 to perform the plurality of vessel assessment tasks. In one embodiment, results of the vessel assessment tasks determined by a machine learning based model trained from clinical reports and results of vessel assessment tasks determined by a machine learning based model trained from manually annotated training data may be combined as multiple evidence for clinical decision making.

In one embodiment, the trained machine learning based model may be continuously fine-tuned based on ground truth data comprising, e.g., clinical reports, annotations, or corrections of the results of the vessel assessment tasks.

Once trained, the trained machine learning based model is applied (e.g., at step 104 of Figure 1) to generate the sets of output embeddings during an online or testing stage.

At step 106 of Figure 1, a plurality of vessel assessment tasks is performed by modelling each of the plurality of sets of output embeddings in a respective probabilistic distribution. In one embodiment, the probabilistic distribution is a probabilistic distribution function that models each of the plurality of sets of output embeddings respectively using a sparse Gaussian process. The sparse Gaussian process is based on inducing points used to approximate the full dataset. The locations and values of the inducing points are learned by maximizing a lower bound on the marginal likelihood, which is known as ELBO (evidence lower bound). The Gaussian process generates a probability distribution over the output where the entropy of this distribution can be used to quantify its uncertainty (e.g., large uncertainties for inputs that are out of training distribution). In other embodiments, the probabilistic distribution is a sigmoid, softmax, or any other activation function.

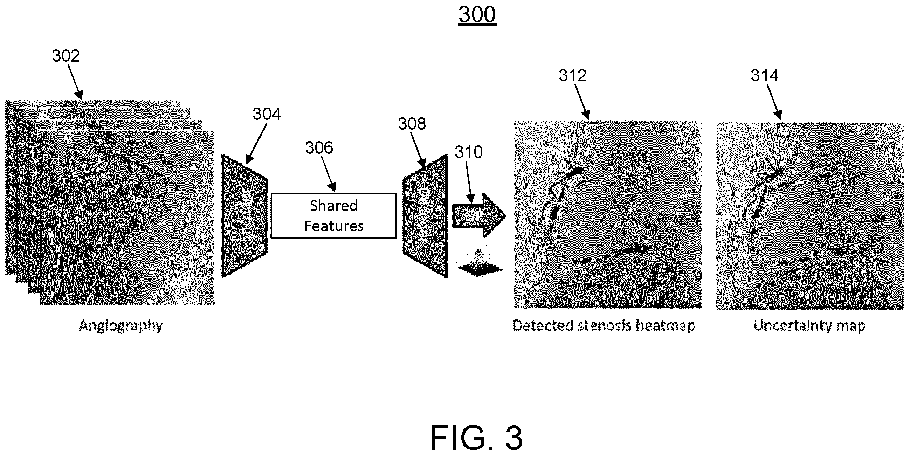

In one embodiment, the Gaussian process also generates a confidence measure for results of the plurality of vessel assessment tasks. The Gaussian process generates a confidence measure that quantifies uncertainty (e.g., as a probability) for the results of the plurality of vessel assessment tasks. The confidence measure may be a confidence measure for the plurality of vessel assessment tasks as a whole, which would indicate a level of consistency between the plurality of vessel assessment tasks. The confidence measure may also be a confidence measure determined for each of the results of the plurality of vessel assessment tasks. The confidence measure may be represented in any suitable form, such as, e.g., a confidence score, a heatmap representing the confidence, etc. The generation of a confidence measure is shown in Figure 3, described in detail below.

The plurality of vessel assessment tasks may be any suitable task for assessing the vessel. In one embodiment, the plurality of vessel assessment tasks includes detection (i.e., localization) of a stenosis in the vessel. In one embodiment, the plurality of vessel assessment tasks includes image-based stenosis grading of a stenosis in the vessel. As used herein, image-based stenosis grading refers to stenosis grading performed directly from the shared features without using results of a segmentation of the lumen of the vessel. The stenosis may be graded or classified as being, e.g., normal, minimal, mild, moderate, severe, or occluded, or may be graded as percent stenosis. In one embodiment, the plurality of vessel assessment tasks includes classification (i.e., labelling) of segments of the vessel. In one embodiment, the plurality of vessel assessment tasks includes global image quality classification. In one embodiment, the plurality of vessel assessment tasks includes detection of poor contrast, branch overlap, and/or foreshortening. In one embodiment, the plurality of vessel assessment tasks includes prior interventions (e.g., stents, bypass grafts, etc.). In one embodiment, the plurality of vessel assessment tasks includes segmentation of the stenosis and/or the vessel.

In one example, as shown in Figure 2, each set of output embeddings output from decoders 210-A, 210-B, and 210-C are modelled as a probabilistic distribution function using Gaussian processes 212-A, 212-B, and 212-C (collectively referred to as Gaussian processes 212) to generate stenosis heatmap 214-A, stenosis grading 214-B, and segment labelling 214-C resulting from the performance of a stenosis detection task, a stenosis grading task, and a segment labelling task, respectively.

At step 108 of Figure 1, results of the plurality of vessel assessment tasks and/or measures of uncertainty of the results of the plurality of vessel assessment tasks are output. For example, the results and/or the measures of uncertainty can be output by displaying the results and/or the measures of uncertainty on a display device of a computer system, storing the results and/or the measures of uncertainty on a memory or storage of a computer system, or by transmitting the results and/or the measures of uncertainty to a remote computer system. The results and/or the measures of uncertainty may be input into other systems, such as, e.g., coronary analysis systems.

The results of the plurality of vessel assessment tasks may be in any suitable form. In one embodiment, the results of the plurality of vessel assessment tasks may comprise one or more heatmaps. For example, the results of the plurality of vessel assessment tasks may comprise a heatmap for each of the plurality of vessel assessment tasks, a composite heatmap that incorporates heatmaps for one or more of the plurality of vessel assessment tasks (e.g., in a weighted manner to filter out specific regions), a heatmap of only disease specific results (e.g., stenosis and/or plaque), a heatmap of only non-disease specific results (e.g., motion artifacts), explicit localization of findings of the vessel assessment tasks on the images (i.e., without heatmaps), disease labels on the images (without heatmaps or explicit location of results), transformation of heatmaps into a set of categories (e.g., SCCT (society of cardiovascular computed tomography) grading scale for stenosis severity), etc.

Advantageously, compared to the conventional sequential combinations of individual single-task networks, the visualization of results and also the intermediate feedback is highly customizable to best fit the end-user. A clinician is not forced to verify validity on fixed single-task result visualizations, but can verify results on dedicated visualizations that are derived from information on all trained vessel assessment tasks based on the latent space shared features.

Figure 3 shows a framework 300 of a multi-task AI system for automatic detection of a stenosis of a vessel and a confidence measure associated with the detection of the stenosis, in accordance with one or more embodiments. In framework 300, encoder 304 receives angiography sequence 302 as input and generates shared features 306 as output. Decoder 308 decodes shared features 306 to generate output embeddings. Gaussian process 310 receives the output embeddings and generates a detected stenosis heatmap 312 and an uncertainty map 314 representing a confidence measure associated with detected stenosis heatmap 312.

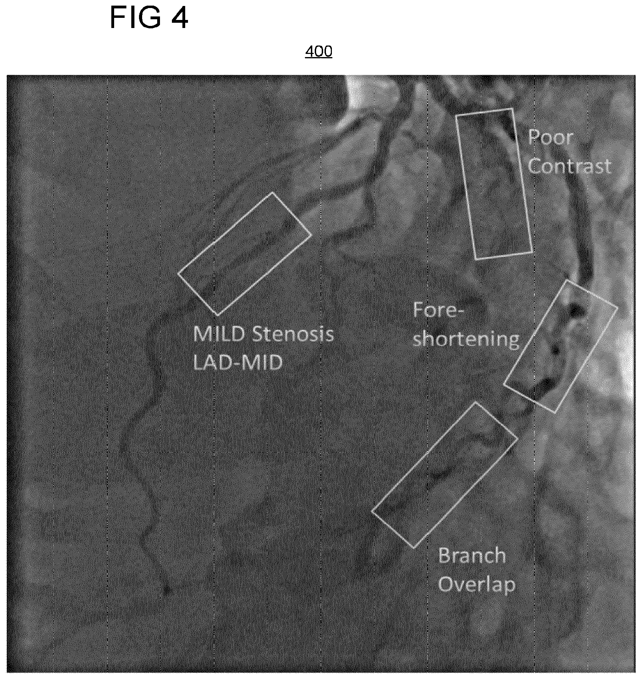

Figure 4 shows an exemplary output image 400 of a multi-task AI system, in accordance with one or more embodiments. In one example, the multi-task AI system is multi-task AI system 204 of Figure 2. Results of vessel assessment tasks performed by the multi-task AI system include localization of a stenosis, poor contrast, foreshortening, branch overlap, and classification of grade (e.g., mild) and segment label (LAD-MID, middle left anterior descending artery) for the stenosis.

Embodiments described herein are described with respect to the claimed systems as well as with respect to the claimed methods. Features, advantages or alternative embodiments herein can be assigned to the other claimed objects and vice versa. In other words, claims for the systems can be improved with features described or claimed in the context of the methods. In this case, the functional features of the method are embodied by objective units of the providing system.

Furthermore, certain embodiments described herein are described with respect to methods and systems utilizing trained machine learning based networks (or models), as well as with respect to methods and systems for training machine learning based networks. Features, advantages or alternative embodiments herein can be assigned to the other claimed objects and vice versa. In other words, claims for methods and systems for training a machine learning based network can be improved with features described or claimed in context of the methods and systems for utilizing a trained machine learning based network, and vice versa.

In particular, the trained machine learning based networks applied in embodiments described herein can be adapted by the methods and systems for training the machine learning based networks. Furthermore, the input data of the trained machine learning based network can comprise advantageous features and embodiments of the training input data, and vice versa. Furthermore, the output data of the trained machine learning based network can comprise advantageous features and embodiments of the output training data, and vice versa.

In general, a trained machine learning based network mimics cognitive functions that humans associate with other human minds. In particular, by training based on training data, the trained machine learning based network is able to adapt to new circumstances and to detect and extrapolate patterns.

In general, parameters of a machine learning based network can be adapted by means of training. In particular, supervised training, semi-supervised training, unsupervised training, reinforcement learning and/or active learning can be used. Furthermore, representation learning (an alternative term is "feature learning") can be used. In particular, the parameters of the trained machine learning based network can be adapted iteratively by several steps of training.

In particular, a trained machine learning based network can comprise a neural network, a support vector machine, a decision tree, and/or a Bayesian network, and/or the trained machine learning based network can be based on k-means clustering, Q-learning, genetic algorithms, and/or association rules. In particular, a neural network can be a deep neural network, a convolutional neural network, or a convolutional deep neural network. Furthermore, a neural network can be an adversarial network, a deep adversarial network and/or a generative adversarial network.

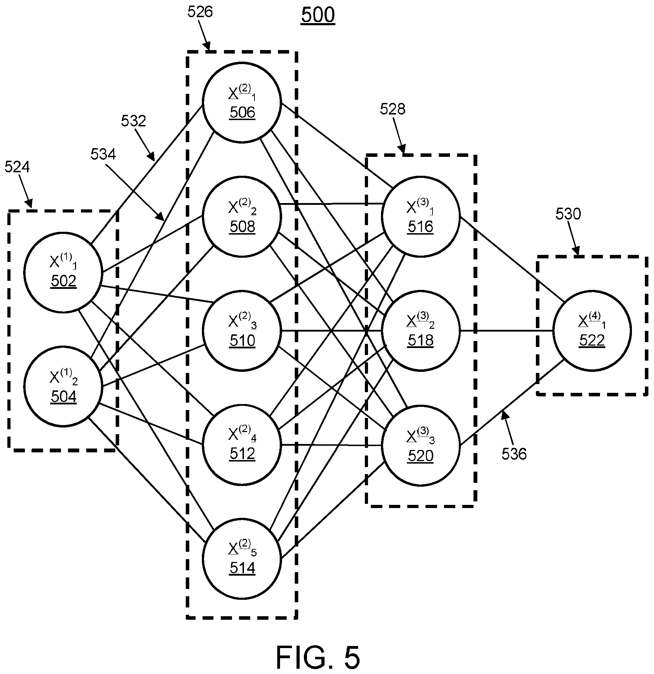

Figure 5 shows an embodiment of an artificial neural network 500, in accordance with one or more embodiments. Alternative terms for "artificial neural network" are "neural network", "artificial neural net" or "neural net". Machine learning networks described herein, such as, e.g., the machine learning based model utilized at step 104 of Figure 1, the multi-task AI system 205 comprising encoder 206 and plurality of decoders 210 of Figure 2, and encoder 304 and decoder 308 of Figure 3, may be implemented using artificial neural network 500.

The artificial neural network 500 comprises nodes 502-522 and edges 532, 534, ..., 536, wherein each edge 532, 534, ..., 536 is a directed connection from a first node 502-522 to a second node 502-522. In general, the first node 502-522 and the second node 502-522 are different nodes 502-522, it is also possible that the first node 502-522 and the second node 502-522 are identical. For example, in Figure 5, the edge 532 is a directed connection from the node 502 to the node 506, and the edge 534 is a directed connection from the node 504 to the node 506. An edge 532, 534, ..., 536 from a first node 502-522 to a second node 502-522 is also denoted as "ingoing edge" for the second node 502-522 and as "outgoing edge" for the first node 502-522.

In this embodiment, the nodes 502-522 of the artificial neural network 500 can be arranged in layers 524-530, wherein the layers can comprise an intrinsic order introduced by the edges 532, 534, ..., 536 between the nodes 502-522. In particular, edges 532, 534, ..., 536 can exist only between neighboring layers of nodes. In the embodiment shown in Figure 5, there is an input layer 524 comprising only nodes 502 and 504 without an incoming edge, an output layer 530 comprising only node 522 without outgoing edges, and hidden layers 526, 528 in-between the input layer 524 and the output layer 530. In general, the number of hidden layers 526, 528 can be chosen arbitrarily. The number of nodes 502 and 504 within the input layer 524 usually relates to the number of input values of the neural network 500, and the number of nodes 522 within the output layer 530 usually relates to the number of output values of the neural network 500.

In particular, a (real) number can be assigned as a value to every node 502-522 of the neural network 500. Here, $x^{(n)}_i$ denotes the value of the i-th node 502-522 of the n-th layer 524-530. The values of the nodes 502-522 of the input layer 524 are equivalent to the input values of the neural network 500, the value of the node 522 of the output layer 530 is equivalent to the output value of the neural network 500. Furthermore, each edge 532, 534, ..., 536 can comprise a weight being a real number, in particular, the weight is a real number within the interval [-1, 1] or within the interval [0, 1]. Here, $w^{(m,n)}_{i,j}$ denotes the weight of the edge between the i-th node 502-522 of the m-th layer 524-530 and the j-th node 502-522 of the n-th layer 524-530. Furthermore, the abbreviation $w^{(n)}_{i,j}$ is defined for the weight $w^{(n,n+1)}_{i,j}$.

In particular, to calculate the output values of the neural network 500, the input values are propagated through the neural network. In particular, the values of the nodes 502-522 of the (n+1)-th layer 524-530 can be calculated based on the values of the nodes 502-522 of the n-th layer 524-530 by

$$ x^{(n+1)}_j = f\left(\sum_i x^{(n)}_i \cdot w^{(n)}_{i,j}\right). $$

Herein, the function f is a transfer function (another term is "activation function"). Known transfer functions are step functions, sigmoid function (e.g. the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 524 are given by the input of the neural network 500, wherein values of the first hidden layer 526 can be calculated based on the values of the input layer 524 of the neural network, wherein values of the second hidden layer 528 can be calculated based in the values of the first hidden layer 526, etc.

In order to set the values $w^{(m,n)}_{i,j}$ for the edges, the neural network 500 has to be trained using training data. In particular, training data comprises training input data and training output data (denoted as $t_i$. For a training step, the neural network 500 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 500 (backpropagation algorithm). In particular, the weights are changed according to

$$ w'^{(n)}_{i,j} = w^{(n)}_{i,j} - \gamma \cdot \delta^{(n)}_j \cdot x^{(n)}_i $$

wherein $\gamma$ is a learning rate, and the numbers $\delta^{(n)}_j$ can be recursively calculated as

$$ \delta^{(n)}_j = \left(\sum_k \delta^{(n+1)}_k \cdot w^{(n+1)}_{j,k}\right) \cdot f'\left(\sum_i x^{(n)}_i \cdot w^{(n)}_{i,j}\right) $$

based on $\delta^{(n+1)}_j$, if the (n+1)-th layer is not the output layer, and

$$ \delta^{(n)}_j = \left(x^{(n+1)}_k - t^{(n+1)}_j\right) \cdot f'\left(\sum_i x^{(n)}_i \cdot w^{(n)}_{i,j}\right) $$

if the (n+1)-th layer is the output layer 530, wherein f is the first derivative of the activation function, and $y^{(n+1)}_j$ is the comparison training value for the j-th node of the output layer 530.

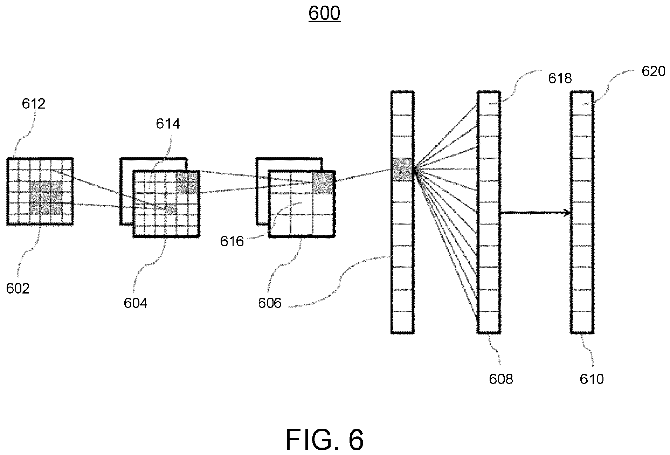

Figure 6 shows a convolutional neural network 600, in accordance with one or more embodiments. Machine learning networks described herein, such as, e.g., the machine learning based model utilized at step 104 of Figure 1, the multi-task AI system 205 comprising encoder 206 and plurality of decoders 210 of Figure 2, and encoder 304 and decoder 308 of Figure 3, may be implemented using convolutional neural network 600.

In the embodiment shown in Figure 6, the convolutional neural network comprises 600 an input layer 602, a convolutional layer 604, a pooling layer 606, a fully connected layer 608, and an output layer 610. Alternatively, the convolutional neural network 600 can comprise several convolutional layers 604, several pooling layers 606, and several fully connected layers 608, as well as other types of layers. The order of the layers can be chosen arbitrarily, usually fully connected layers 608 are used as the last layers before the output layer 610.

In particular, within a convolutional neural network 600, the nodes 612-620 of one layer 602-610 can be considered to be arranged as a d-dimensional matrix or as a d-dimensional image. In particular, in the two-dimensional case the value of the node 612-620 indexed with i and j in the n-th layer 602-610 can be denoted as $x^{(n)}_{[i,j]}$. However, the arrangement of the nodes 612-620 of one layer 602-610 does not have an effect on the calculations executed within the convolutional neural network 600 as such, since these are given solely by the structure and the weights of the edges.

In particular, a convolutional layer 604 is characterized by the structure and the weights of the incoming edges forming a convolution operation based on a certain number of kernels. In particular, the structure and the weights of the incoming edges are chosen such that the values $x^{(n)}_k$ of the nodes 614 of the convolutional layer 604 are calculated as a convolution $x^{(n)}_k = K_k * x^{(n-1)}$ based on the values $x^{(n-1)}$ of the nodes 612 of the preceding layer 602, where the convolution * is defined in the two-dimensional case as

$$x^{(n)}_k[i,j] = \left(K_k * x^{(n-1)}\right)[i,j] = \sum_{i'}\sum_{j'} K_k[i',j'] \cdot x^{(n-1)}[i-i', j-j'].$$

Here the k-th kernel $K_k$ is a d-dimensional matrix (in this embodiment a two-dimensional matrix), which is usually small compared to the number of nodes 612-618 (e.g. a 3x3 matrix, or a 5x5 matrix). In particular, this implies that the weights of the incoming edges are not independent, but chosen such that they produce said convolution equation. In particular, for a kernel being a 3x3 matrix, there are only 9 independent weights (each entry of the kernel matrix corresponding to one independent weight), irrespectively of the number of nodes 612-620 in the respective layer 602-610. In particular, for a convolutional layer 604, the number of nodes 614 in the convolutional layer is equivalent to the number of nodes 612 in the preceding layer 602 multiplied with the number of kernels.

If the nodes 612 of the preceding layer 602 are arranged as a d-dimensional matrix, using a plurality of kernels can be interpreted as adding a further dimension (denoted as "depth" dimension), so that the nodes 614 of the convolutional layer 604 are arranged as a (d+1)-dimensional matrix. If the nodes 612 of the preceding layer 602 are already arranged as a (d+1)-dimensional matrix comprising a depth dimension, using a plurality of kernels can be interpreted as expanding along the depth dimension, so that the nodes 614 of the convolutional layer 604 are arranged also as a (d+1)-dimensional matrix, wherein the size of the (d+1)-dimensional matrix with respect to the depth dimension is by a factor of the number of kernels larger than in the preceding layer 602.

The advantage of using convolutional layers 604 is that spatially local correlation of the input data can exploited by enforcing a local connectivity pattern between nodes of adjacent layers, in particular by each node being connected to only a small region of the nodes of the preceding layer.

In embodiment shown in Figure 6, the input layer 602 comprises 36 nodes 612, arranged as a two-dimensional 6x6 matrix. The convolutional layer 604 comprises 72 nodes 614, arranged as two two-dimensional 6x6 matrices, each of the two matrices being the result of a convolution of the values of the input layer with a kernel. Equivalently, the nodes 614 of the convolutional layer 604 can be interpreted as arranges as a three-dimensional 6x6x2 matrix, wherein the last dimension is the depth dimension.

A pooling layer 606 can be characterized by the structure and the weights of the incoming edges and the activation function of its nodes 616 forming a pooling operation based on a non-linear pooling function f. For example, in the two dimensional case the values $x^{(n)}$ of the nodes 616 of the pooling layer 606 can be calculated based on the values $x^{(n-1)}$ of the nodes 614 of the preceding layer 604 as

$$x^{(n)}[i,j] = f(x^{(n-1)}[id_1, jd_2], \ldots, x^{(n-1)}[id_1+d_1-1, jd_2+d_2-1])$$

In other words, by using a pooling layer 606, the number of nodes 614, 616 can be reduced, by replacing a number $d1 \cdot d2$ of neighboring nodes 614 in the preceding layer 604 with a single node 616 being calculated as a function of the values of said number of neighboring nodes in the pooling layer. In particular, the pooling function f can be the max-function, the average or the L2-Norm. In particular, for a pooling layer 606 the weights of the incoming edges are fixed and are not modified by training.

The advantage of using a pooling layer 606 is that the number of nodes 614, 616 and the number of parameters is reduced. This leads to the amount of computation in the network being reduced and to a control of overfitting.

In the embodiment shown in Figure 6, the pooling layer 606 is a max-pooling, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

A fully-connected layer 608 can be characterized by the fact that a majority, in particular, all edges between nodes 616 of the previous layer 606 and the nodes 618 of the fully-connected layer 608 are present, and wherein the weight of each of the edges can be adjusted individually.

In this embodiment, the nodes 616 of the preceding layer 606 of the fully-connected layer 608 are displayed both as two-dimensional matrices, and additionally as non-related nodes (indicated as a line of nodes, wherein the number of nodes was reduced for a better presentability). In this embodiment, the number of nodes 618 in the fully connected layer 608 is equal to the number of nodes 616 in the preceding layer 606. Alternatively, the number of nodes 616, 618 can differ.

Furthermore, in this embodiment, the values of the nodes 620 of the output layer 610 are determined by applying the Softmax function onto the values of the nodes 618 of the preceding layer 608. By applying the Softmax function, the sum the values of all nodes 620 of the output layer 610 is 1, and all values of all nodes 620 of the output layer are real numbers between 0 and 1.

A convolutional neural network 600 can also comprise a ReLU (rectified linear units) layer or activation layers with non-linear transfer functions. In particular, the number of nodes and the structure of the nodes contained in a ReLU layer is equivalent to the number of nodes and the structure of the nodes contained in the preceding layer. In particular, the value of each node in the ReLU layer is calculated by applying a rectifying function to the value of the corresponding node of the preceding layer.

The input and output of different convolutional neural network blocks can be wired using summation (residual / dense neural networks), element-wise multiplication (attention) or other differentiable operators. Therefore, the convolutional neural network architecture can be nested rather than being sequential if the whole pipeline is differentiable.

In particular, convolutional neural networks 600 can be trained based on the backpropagation algorithm. For preventing overfitting, methods of regularization can be used, e.g. dropout of nodes 612-620, stochastic pooling, use of artificial data, weight decay based on the L1 or the L2 norm, or max norm constraints. Different loss functions can be combined for training the same neural network to reflect the joint training objectives. A subset of the neural network parameters can be excluded from optimization to retain the weights pretrained on another datasets.

Systems, apparatuses, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, apparatus, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, apparatus, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figure 1. Certain steps or functions of the methods and workflows described herein, including one or more of the steps or functions of Figure 1, may be performed by a server or by another processor in a network-based cloud-computing system. Certain steps or functions of the methods and workflows described herein, including one or more of the steps of Figure 1, may be performed by a client computer in a network-based cloud computing system. The steps or functions of the methods and workflows described herein, including one or more of the steps of Figure 1, may be performed by a server and/or by a client computer in a network-based cloud computing system, in any combination.

Systems, apparatus, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figure 1, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

A high-level block diagram of an example computer 702 that may be used to implement systems, apparatus, and methods described herein is depicted in Figure 7. Computer 702 includes a processor 704 operatively coupled to a data storage device 712 and a memory 710. Processor 704 controls the overall operation of computer 702 by executing computer program instructions that define such operations. The computer program instructions may be stored in data storage device 712, or other computer readable medium, and loaded into memory 710 when execution of the computer program instructions is desired. Thus, the method and workflow steps or functions of Figure 1 can be defined by the computer program instructions stored in memory 710 and/or data storage device 712 and controlled by processor 704 executing the computer program instructions. For example, the computer program instructions can be implemented as computer executable code programmed by one skilled in the art to perform the method and workflow steps or functions of Figure 1. Accordingly, by executing the computer program instructions, the processor 704 executes the method and workflow steps or functions of Figure 1. Computer 702 may also include one or more network interfaces 706 for communicating with other devices via a network. Computer 702 may also include one or more input/output devices 708 that enable user interaction with computer 702 (e.g., display, keyboard, mouse, speakers, buttons, etc.).

Processor 704 may include both general and special purpose microprocessors, and may be the sole processor or one of multiple processors of computer 702. Processor 704 may include one or more central processing units (CPUs), for example. Processor 704, data storage device 712, and/or memory 710 may include, be supplemented by, or incorporated in, one or more applicationspecific integrated circuits (ASICs) and/or one or more field programmable gate arrays (FPGAs).

Data storage device 712 and memory 710 each include a tangible non-transitory computer readable storage medium. Data storage device 712, and memory 710, may each include high-speed random access memory, such as dynamic random access memory (DRAM), static random access memory (SRAM), double data rate synchronous dynamic random access memory (DDR RAM), or other random access solid state memory devices, and may include non-volatile memory, such as one or more magnetic disk storage devices such as internal hard disks and removable disks, magneto-optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

Input/output devices 708 may include peripherals, such as a printer, scanner, display screen, etc. For example, input/output devices 708 may include a display device such as a cathode ray tube (CRT) or liquid crystal display (LCD) monitor for displaying information to the user, a keyboard, and a pointing device such as a mouse or a trackball by which the user can provide input to computer 702.

An image acquisition device 714 can be connected to the computer 702 to input image data (e.g., medical images) to the computer 702. It is possible to implement the image acquisition device 714 and the computer 702 as one device. It is also possible that the image acquisition device 714 and the computer 702 communicate wirelessly through a network. In a possible embodiment, the computer 702 can be located remotely with respect to the image acquisition device 714.

Any or all of the systems and apparatus discussed herein may be implemented using one or more computers such as computer 702.

One skilled in the art will recognize that an implementation of an actual computer or computer system may have other structures and may contain other components as well, and that Figure 7 is a high level representation of some of the components of such a computer for illustrative purposes.

The foregoing Detailed Description is to be understood as being in every respect illustrative and exemplary, but not restrictive, and the scope of the invention disclosed herein is not to be determined from the Detailed Description, but rather from the claims as interpreted according to the full breadth permitted by the patent laws. It is to be understood that the embodiments shown and described herein are only illustrative of the principles of the present invention and that various modifications may be implemented by those skilled in the art without departing from the scope of the invention. Those skilled in the art could implement various other feature combinations without departing from the scope of the invention.

**Claims**

1. A computer-implemented method comprising:

   receiving (102) a temporal sequence of medical images (202, 302) of a vessel of a patient;
   generating (104) a plurality of sets of output embeddings using a machine learning based model (206 and 210, 304 and 308) trained using multi-task learning, the plurality of sets of output embeddings generated based on shared features (208, 306) extracted from the temporal sequence of medical images;
   performing (106) a plurality of vessel assessment tasks by modelling each of the plurality of sets of output embeddings in a respective probabilistic distribution; and
   outputting (108) results (214-A, 214-B, 214-C, and 312) of the plurality of vessel assessment tasks.

2. The computer-implemented method of claim 1, wherein the temporal sequence of medical images is an angiography sequence; and/or wherein the temporal sequence of medical images is acquired at a plurality of different acquisition angles.

3. The computer-implemented method of claim 1 or 2, wherein generating a plurality of sets of output embeddings using a machine learning based model trained using multi-task learning comprises:

   extracting shared features from the temporal sequence of medical images using an encoder network (206, 304); and
   decoding the shared features using a plurality of decoders (210, 308) to respectively generate the plurality of sets of output embeddings.

4. The computer-implemented method of any one of claims 1-3, wherein performing a plurality of vessel assessment tasks by modelling each of the plurality of sets of output embeddings in a respective probabilistic distribution comprises:
   modelling each of the plurality of sets of output embeddings in a respective Gaussian process (212, 310).

5. The computer-implemented method of claim 4, wherein performing a plurality of vessel assessment tasks by modelling each of the plurality of sets of output embeddings in a respective probabilistic distribution comprises:
   determining a confidence measure (314) for the results of the plurality of vessel assessment tasks using the Gaussian process.

6. The computer-implemented method of claim 1, wherein the plurality of vessel assessment tasks comprises at least one of:

   localization of a stenosis in the vessel;
   image-based stenosis grading of a stenosis in the vessel; and
   segment labelling of a stenosis in the vessel and segmentation of the stenosis.

7. An apparatus comprising:

   means for receiving (102) a temporal sequence of medical images (202, 302) of a vessel of a patient;
   means for generating (104) a plurality of sets of output embeddings using a machine learning based model (206 and 210, 304 and 308) trained using multi-task learning, the plurality of sets of output embeddings generated based on shared features (208, 306) extracted from the temporal sequence of medical images;
   means for performing (106) a plurality of vessel assessment tasks by modelling each of the plurality of sets of output embeddings in a respective probabilistic distribution; and
   means for outputting (108) results (214-A, 214-B, 214-C, and 312) of the plurality of vessel assessment tasks.

8. The apparatus of claim 7, wherein the means for generating a plurality of sets of output embeddings using a machine learning based model trained using multi-task learning comprises:

   means for extracting shared features from the temporal sequence of medical images using an encoder network (206, 304); and
   means for decoding the shared features using a plurality of decoders (210, 308) to respectively generate the plurality of sets of output embeddings.

9. The apparatus of claim 7 or 8, wherein the means for performing a plurality of vessel assessment tasks by modelling each of the plurality of sets of output embeddings in a respective probabilistic distribution comprises:
means for modelling each of the plurality of sets of output embeddings in a respective Gaussian process (212, 310).

10. The apparatus of claim 9, wherein the means for performing a plurality of vessel assessment tasks by modelling each of the plurality of sets of output embeddings in a respective probabilistic distribution further comprises:
means for determining a confidence measure (314) for the results of the plurality of vessel assessment tasks using the Gaussian process.

11. The apparatus of any one of claims 7 - 10, wherein the means are implemented to collaboratively execute the method of any one of claims 1-6.

12. A non-transitory computer readable medium storing computer program instructions, the computer program instructions when executed by a processor cause the processor to perform operations comprising:

receiving (102) a temporal sequence of medical images (202, 302) of a vessel of a patient;
generating (104) a plurality of sets of output embeddings using a machine learning based model (206 and 210, 304 and 308) trained using multi-task learning, the plurality of sets of output embeddings generated based on shared features (208, 306) extracted from the temporal sequence of medical images;
performing (106) a plurality of vessel assessment tasks by modelling each of the plurality of sets of output embeddings in a respective probabilistic distribution; and
outputting (108) results (214-A, 214-B, 214-C, and 312) of the plurality of vessel assessment tasks.

13. The non-transitory computer readable medium of claim 12, wherein the computer program instructions when executed by a processor cause the processor to perform operations comprising the steps of the method according to any one of claims 1-6.

# FIG 1

100

Receive a temporal sequence of medical images of a vessel of a patient
102

Generate a plurality of sets of output embeddings using a machine
learning based model trained using multi-task learning, the plurality of
sets of output embeddings generated based on shared features extracted
from the temporal sequence of medical images
104

Perform a plurality of vessel assessment tasks by modelling each of the
plurality of sets of output embeddings in a respective probabilistic
distribution
106

Output results of the plurality of vessel assessment tasks
108

FIG. 2

EP 4 343 782 A1

FIG. 3

# FIG 4

400

FIG. 5

500

530

536

528

526

532

534

524

$X^{(4)}_1$ 522

$X^{(3)}_1$ 516    $X^{(3)}_2$ 518    $X^{(3)}_3$ 520

$X^{(2)}_1$ 506    $X^{(2)}_2$ 508    $X^{(2)}_3$ 510    $X^{(2)}_4$ 512    $X^{(2)}_5$ 514

$X^{(1)}_1$ 502    $X^{(1)}_2$ 504

600

FIG. 6

# FIG 7

702

Network interface
706

I/O
708

Processor
704

Storage
712

Memory
710

Image Acquisition Device
714

Europäisches
Patentamt

European
Patent Office

Office européen
des brevets

# EUROPEAN SEARCH REPORT

Application Number

EP 23 19 8282

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 4 057 221 A1 (SIEMENS HEALTHCARE GMBH [DE]) 14 September 2022 (2022-09-14) * paragraphs [0012] – [0038]; figures 2,4 * | 1-13 | INV. G16H30/40 G06N3/045 G16H50/20 |
| A | FRANCESCO PAOLO CASALE ET AL: "Gaussian Process Prior Variational Autoencoders", ARXIV.ORG, CORNELL UNIVERSITY LIBRARY, 201 OLIN LIBRARY CORNELL UNIVERSITY ITHACA, NY 14853, 28 October 2018 (2018-10-28), XP081044398, * the whole document * | 1-13 | |
| A | XIAO LIU ET AL: "Learning Disentangled Representations in the Imaging Domain", ARXIV.ORG, CORNELL UNIVERSITY LIBRARY, 201 OLIN LIBRARY CORNELL UNIVERSITY ITHACA, NY 14853, 29 July 2022 (2022-07-29), XP091282977, DOI: 10.1016/J.MEDIA.2022.102516 * the whole document * | 1-13 | |

**TECHNICAL FIELDS SEARCHED (IPC)**

G16H
G06N

The present search report has been drawn up for all claims

| Place of search | Date of completion of the search | Examiner |
|---|---|---|
| Munich | 8 February 2024 | Díaz de Lezana, C |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 23 19 8282

This annex lists the patent family members relating to the patent documents cited in the above-mentioned European search report.
The members are as contained in the European Patent Office EDP file on
The European Patent Office is in no way liable for these particulars which are merely given for the purpose of information.

08-02-2024

| Patent document cited in search report | | Publication date | Patent family member(s) | | Publication date |
|---|---|---|---|---|---|
| EP 4057221 | A1 | 14-09-2022 | CN 115049582 | A | 13-09-2022 |
| | | | EP 4057221 | A1 | 14-09-2022 |

EPO FORM P0459

For more details about this annex : see Official Journal of the European Patent Office, No. 12/82